# EUROPÄISCHE PATENTSCHRIFT

(11) **EP 1 219 169 B1**
(45) Veröffentlichungstag und Bekanntmachung des Hinweises auf die Patenterteilung: **01.10.2003**
(21) Anmeldenummer: 01100201.1
(22) Anmeldetag: 01.01.2001
(51) Int. Cl.: A01K 11/00, A61B 10/00, G01N 1/04

(54) **Modifizierte Ohrmarken und Verfahren zur Gewebeentnahme**
Modified ear tag and method for taking a tissue sample
Marque d'oreille modifiée et procédé de prélèvement d'echantillon de tissu

(43) Veröffentlichungstag der Anmeldung: 03.07.2002
(73) Patentinhaber: Agrobiogen GmbH Biotechnologie, Larezhausen, 86567 Hilgertshausen (DE)
(72) Erfinder: Brem, Gottfried, Prof. Dr. Dr., Larezhausen, 86567 Hilgertshausen (DE)
(74) Vertreter: Becker Kurig Straus

(56) Entgegenhaltungen:
- EP-A- 1 060 662
- WO-A-99/61882
- DE-A- 19 740 429

## Beschreibung

Die vorliegende Erfindung betrifft modifizierte Ohrmarken, die eine Dornplatte, eine Gegenplatte sowie einen Probenaufnahmebehälter und Mittel zum Gewinnen der Probe umfassen, wobei das Mittel zum Gewinnen der Probe eine lösbare Hohlspitze aufweist und die Gegenplatte keine zur Durchführung des Dorns ausgebildete Durchgangsöffnung besitzt. Die Erfindung betrifft insbesondere die Verwendung dieser Ohrmarken zur Kennzeichnung von Tieren mit gleichzeitiger Entnahme einer biologischen Probe sowie ein Verfahren zur Kennzeichnung von Tieren mit diesen Ohrmarken.

Zur Markierung und Identifizierung von Tieren werden seit langem Ohrmarken genutzt, wobei sich in den letzten Jahren flexible Ohrmarken aus Kunststoff, meist Polyurethan oder -ethylen, durchgesetzt haben. Alle diese Ohrmarken weisen eine Dornplatte, auch männliches Teil oder männliche Flagge genannt und ein weibliches Teil, die Gegenplatte oder weibliche Flagge, auf. Die Dornplatte besitzt gewöhnlich einen Dorn, der eine Spitze aus Metall oder hartem Kunststoff trägt. Die Gegenplatte umfaßt eine Öffnung, durch die der Dorn nach dem Durchdringen des Ohres beim Schliessen der Ohrmarke gedrückt wird, so dass die beiden Ohrmarkenteile miteinander verbunden werden. Beim Einziehen konventioneller Ohrmarken wird das Ohr mit der scharfen Spitze des Dorns durchtstoßen, d. h. die Spitze dringt durch die Haut, den Knorpel und die Haut auf der gegenüberliegenden Seite des Ohres.

In der Regel werden von der Spitze des Dorns der Ohrmarke Haut und Knorpel punktförmig durchstossen und dann in Form eines längsförmigen Risses so weit aufgetrennt, dass der gesamte Kopf des Dorns durchschlüpfen kann. Dies hat zur Folge, dass der Dorn der eingezogenen Ohrmarke von dem auseinandergedrängten Ohrgewebe, das sich elastisch schliesst, mehr oder weniger fest umgeben wird. Ein Nachteil dabei besteht darin, dass durch Dreh- und Ziehbewegungen der Ohrmarke in der Wunde die Wundränder stark gereizt werden und die Einheilung behindert wird. Durch die vorhandenen oder eingedrungenen Bakterien kommt es darüber hinaus häufig zu Schwellungen, Vereiterungen mit Sekretstau und Entwicklung von wucherndem Granulationsgewebe.

Seit kurzem werden Ohrmarken auch dazu verwendet, gleichzeitig mit dem Einziehen der Ohrmarke eine Gewebeprobe für eine spätere DNA-Analyse zu gewinnen. Ein Verfahren der Gewebeprobeentnahme in nummerierte Probenkapseln ist beispielsweise in der DE 197 40 429 A1 beschrieben. Bei diesem Verfahren werden die Nummern der verwendeten Ohrmarken und die Nummern des Probenbehälters auf elektronischem Weg miteinander verknüpft.

In PCT/EP98/03075 wird weiter eine Ohrmarke beschrieben, bei der Ohrmarke und Sammelbehälter vor der Ausgabe an die Nutzer mittels Laser oder anderer Technologien mit gleicher Codierung und der gleichen Tieridentifikationsnummer versehen werden. Dadurch ist eine fehler- und verwechslungsfreie Identitäts-Verknüpfung von Ohrmarke und Gewebeprobe-Sammelbehälter gewährleistet. Die Hohlspitze des Dorns, die mit dem Dorn über eine Sollbruchstelle verbunden ist, nimmt im Ohr eine Probe auf, transportiert diese automatisch in den Sammelbehälter, trennt sich vom Dorn und verschliesst die Probe luftdicht im Sammelbehälter.

Der Probensammelbehälter wird üblicherweise nach dem Einziehen der Ohrmarke entnommen und eingesammelt. Er kann jedoch auch problemlos an der Ohrmarke verhaften und damit beim Tier bleiben, wenn dies gewünscht wird. Der Vorgang des Einziehens der Ohrmarken und des Gewinnens der Gewebeprobe sind somit unabhängig voneinander. Die Einsammlung der Gewebeprobe kann im Prinzip auch noch Jahre nach dem Einziehen der Ohrmarke erfolgen, indem erst zu diesem Zeitpunkt der Probensammelbehälter von der weiblichen Flagge der Ohrmarke getrennt wird.

In der Europäischen Union ist per Rechtsverordnung vorgeschrieben, dass alle geborenen Kälber innerhalb der ersten Lebenswoche zwei Ohrmarken erhalten müssen. Werden nun für diese Kennzeichnung die in der PCT/EP98/03075 beschriebenen Ohrmarken verwendet, dann könnte beispielsweise der Sammelbehälter der einen Ohrmarke sofort nach dem Einziehen der Ohrmarke entnommen werden. Der Sammelbehälter der zweiten Ohrmarke hingegen verbleibt - verbunden mit der weiblichen Flagge - am Tier. Zu einem späteren Zeitpunkt, z. B. beim Export, Schlachten oder Zerlegen des Tieres wird dann der zweite noch am Tier vorhandene Probensammelbehälter, der seit dem Einziehen der Ohrmarke eine Probe enthält, entnommen und zur Analyse ins Labor geschickt. Dieses Vorgehen erspart bei der Überprüfungsprobe das Gewinnen und Kennzeichnen einer neuen Probe und ermöglicht den Erhalt einer unverwechselbaren, da gleichzeitig mit der Ohrmarke gekennzeichneten Zweitprobe, die zwar bereits am Beginn des Lebens des Tieres entnommen, jedoch im Probenaufnahmebehälter 1 konserviert und am Tier verblieben ist und beispielsweise erst am Ende des Lebens des Tieres genutzt wird.

Es hat sich gezeigt, dass es bei neugeborenen Tieren bestimmter Spezies vorkommen kann, dass sich eine Probenentnahme nicht mit der gewünschten Zuverlässigkeit bewerkstelligen lässt. Bei sehr jungen Tieren, insbesondere wenn es sich um Lämmer oder Ferkel handelt, ist das gesamte Ohr so weich, dass es beim Stech/Stanzvorgang zuwenig Widerstand bietet. Bei ungeschickter Vorgehensweise wird beim Einziehen der Ohrmarken in der Folge dessen Ohrgewebe zerrissen und nicht sauber durchstochen/gestanzt. In diesen Fällen kommt es dann nicht immer zur Entnahme/Gewinnung einer Gewebeprobe. Die Funktion der Ohrmarke an sich ist davon nicht beeinträchtigt.

Eine Aufgabe der vorliegenden Erfindung war es daher die vorstehenden Nachteile des Standes der Technik zu überwinden.

Diese Aufgabe wurde gelöst durch eine modifizierte Ohrmarke, welche eine Dornplatte 10 umfasst, eine Gegenplatte 11 sowie einen Probenaufnahmebehälter 1 und Mittel zum Gewinnen der Probe 4, das nach Gewinnen der Probe in den Probenaufnahmebehälter eingeführt wird und diesen dichtend verschließt, wobei das Mittel zum Gewinnen der Probe 4 eine Hohlspitze aufweist und die Gegenplatte 11 keine zur Durchführung des Dorns ausgebildete Durchgangsöffnung besitzt.

In den Figuren,

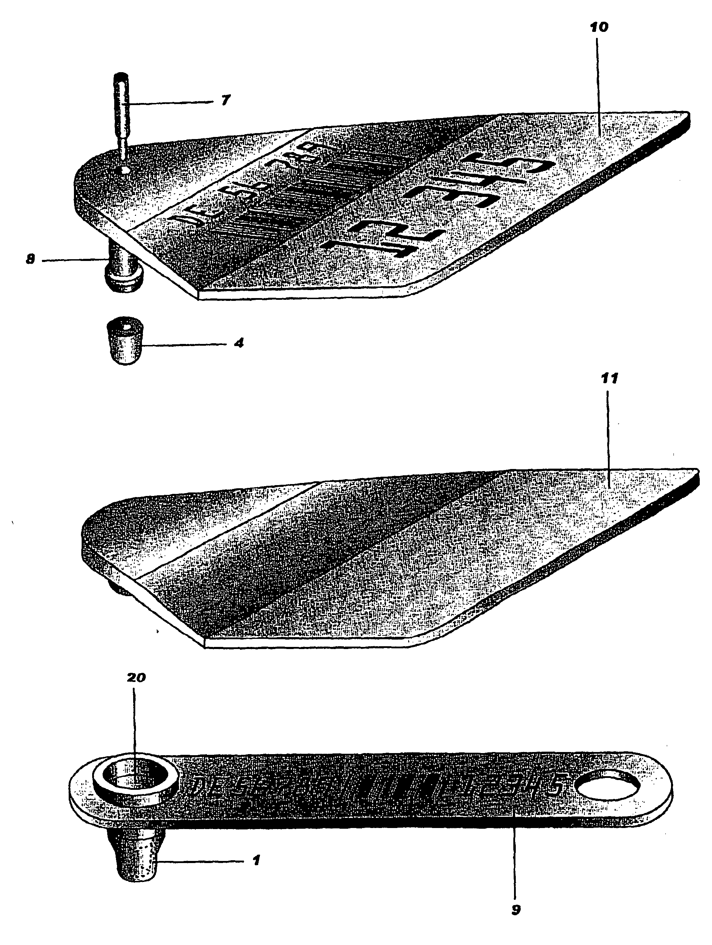

Fig. 1 zeigt ein Funktionsbild von Ohrmarkenteilen, Probengewinnungsmittel 4 und Probenaufnahmebehälter 1;
Fig. 2 zeigt schematisch einen Stanzvorgang beim Einziehen einer Ohrmarke;

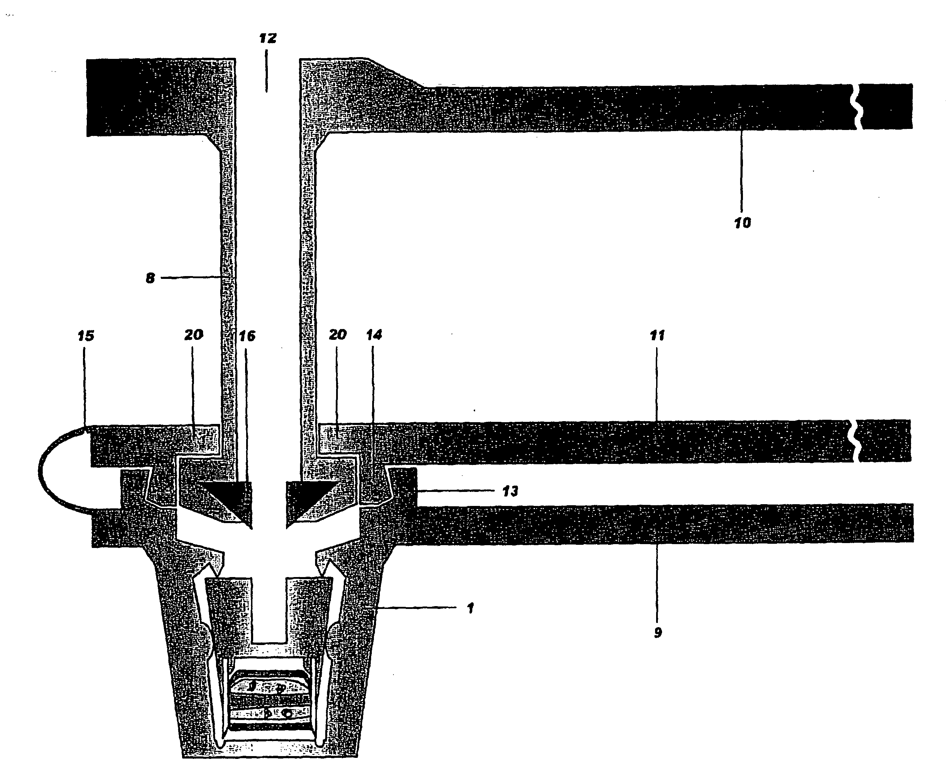

Fig. 3 ist eine Querschnittsansicht von Ohrmarkenteilen, Probenaufnahmebehälter 1 und Probengewinnungsmittel 4 unmittelbar nach dem Schliessen der Ohrmarke und Gewinnung der Probe;

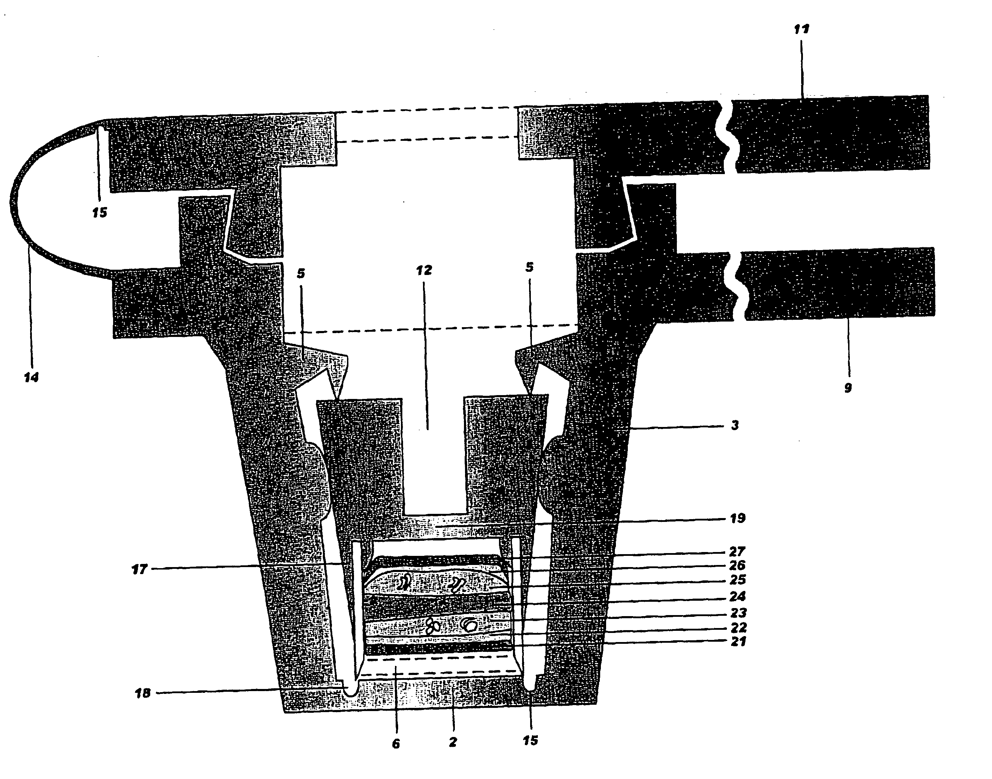

Fig. 4 ist eine vergrößerte Ansicht von Fig. 3 mit Probenaufnahmebehälter 1 und Probengewinnungsmittel 4 mit gestanzter und verpackter Probe.

In den Figuren bezeichnen :
- 1: Probenaufnahmebehälter
- 2: Bodenteil von 1
- 3: Seitenwände von 1
- 4: Probengewinnungsmittel
- 5: Rückhaltevorrichtung für 4
- 6: Probenraum
- 7: Vorderende von 4
- 8: Dorn
- 9: Lasche
- 10: Dornplatte der Ohrmarke (männlicher Teil)
- 11: Gegenplatte (Lochplatte) der Ohrmarke (weiblicher Teil)
- 12: Innenbohrung von 4 und 8
- 13: Positionierungsring von 1
- 14: Positionierungsring von 11
- 15: Verbindungsstege zwischen 1 und 11
- 16: Sollbruchstelle
- 17: Metallhülse in der Dornspitze
- 18: Ringnut als Sollbruchstelle in 2
- 19: Zwischenboden in 4
- 20: Rückhalteschulter für Dombasis
- 21: Epidermis (Oberhaut) - Ohrinnenseite
- 22: Korium (Lederhaut)
- 23: Subcutis (Unterhaut)
- 24: Ohrknorpel
- 25: Subcutis (Unterhaut)
- 26: Korium (Lederhaut)
- 27: Epidermis (Oberhaut) - Ohraussenseite

Durch die vorstehend aufgeführten Modifikationen beim Aufbau der Ohrmarken wird es möglich, die im Stand der Technik bekannten Nachteile entweder ganz zu vermeiden (bei der Probenentnahme) oder zumindest stark zu verringern (bei der Einheilung).

Bei der erfindungsgemäßen Ohrmarke ist die Spitze des bei den bekannten Ohrmarken eingesetzten Dorns durch eine Hohlspitze mit einer Spitzenöffnung ersetzt, deren Durchmesser vorzugsweise im wesentlichen dem Durchmesser des Dorns entspricht. Diese Hohlspitze ist vorzugsweise aus einem harten Material gefertigt, wie Metall, das einen geschliffenen Rand aufweisen kann, oder Glas, Keramik, hartem Kunststoff oder einem ähnlichem Material, oder besitzt ein angepaßtes Einlegeteil aus einem dieser Materialien. Gemäß eine bevorzugten Ausführungsform weist der Schneiderand der Hohlspitze einen wellenförmigen Verlauf auf, so dass der erste Kontakt zwischen Rand und Ohr an zwei oder mehr Stellen punktförmig auftritt. An diesen Punkten wird die Epidermis sehr leicht durchdrungen und durch weiteren Druck und Eindringen der Spitze gelangt dann das gesamte Rund der Hohlöffnung in und durch das Ohr. In diesem Fall ist für das Durchdringen weniger Kraft erforderlich, da der Stanzvorgang zumindest teilweise durch eine Art Schneidevorgang ersetzt wird.

Die Form einer Hohlspitze erlaubt es zudem in den Boden der Hohlspitze ein von außen identifizierbares/erkennbares Kennzeichnungsmittel, wie einen elektronischen Chip o. ä., einzulegen und dort zu fixieren. Beim Einziehen der Ohrmarke und dem Gewinnen der Gewebeprobe wird dieses Kennzeichnungsmittel/der Chip zusammen mit der Gewebeprobe automatisch in den Probenaufnahmebehälter 1 überführt und ist dazu geeignet, dort die Probe zu kennzeichnen. Die elektronische Kennung auf dem Chip in der Dornspitze ist identisch mit der elektronischen Kennung des Chips, der auf der Ohrmarke fixiert bzw. aufgeschweisst ist. Damit sind Probe, Probenaufnahmebehälter 1 sowie Dornspitze und Ohrmarke eindeutig elektronisch gekennzeichnet, ohne dass eine Verknüpfung von Daten, beispielsweise elektronisch über EDV, notwendig wäre.

Damit nach dem Beschriften Ohrmarke und Sammelbehälter zuverlässig zusammen bleiben, ist der Probenaufnahmebehälter 1 mit der Gegenplatte 11 der Ohrmarke mit mindestens einem Steg 15 verbunden. Diese Verbindung weist vorzugsweise eine Sollbruchstelle auf, die derart ausgestaltet ist, dass beim Trennen des Probenaufnahmebehälters 1 von der Gegenplatte 11 der Ohrmarke der Steg am Probenaufnahmebehälter 1 und nicht an der Flagge 11 verbleibt.

Die männliche Flagge bzw. Dornplatte 10 und weibliche Flagge bzw. Gegenplatte 11 können an der Unterkante durch einen schmalen Steg aus Kunststoff miteinander verbunden sein, so dass die Ohrmarke zusammen mit dem Probenbehälter eine Einheit bildet, die in einem Arbeitsgang beschriftet und dann für die Auslieferung zusammengeklappt wird. Vor dem Einziehen der Ohrmarken können beim Einlegen der Ohrmarkenteile in die Zange die beiden Flaggen durch Abreissen voneinander getrennt werden.

Wenn die Verbindung zwischen den beiden Flaggen nicht gelöst wird, bleiben bei der eingezogenen Ohrmarke die Flaggen 10, 11 in Verbindung. Dies führt dazu, dass sich beispielsweise bei Weideführung der Tiere keine Drähte oder Schnüre um die Ohrmarken bzw. den Dorn der Ohrmarken wickeln können, da die eingezogene Ohrmarke einen geschlossenen Ring darstellt. Damit wird eine häufige Ursache für das Ausreissen von Ohrmarken vermieden.

Die Gegenplatte der Ohrmarke enthält keine Öffnung zur Einführung des Dorns und unterscheidet sich damit von allen bekannten Ohrmarken, die durchwegs eine Öffnung in der Gegenplatte aufweisen. Zur Erleichterung der Anbringung der Ohrmarke kann an der Stelle, an der der Dorn 8 durch die Gegenplatte 11 hindurchtreten soll, eine Markierung vorgesehen sein, die es erlaubt, festzustellen, wo der Dorn die weibliche Flagge treffen und durchdringen soll. Als Beispiel für derartige Markierungen können ein Kreuz oder auch ein kleine Vertiefung genannt werden, wobei derartige Maßnahmen auch die Einführung bzw. Durchführung des Dorns 8 durch die weibliche Flagge 11 erleichtern.

Der Probenaufnahmebehälter 1 ist vorzugsweise durch eine mit Ultraschall aufgeschweisste oder aufgeklebte Folie oder durch eine andere Vorrichtung luft- und wasserdicht abgeschlossen.

Darüber hinaus sind die weibliche Flagge 11 und der Probenaufnahmebehälter 1 über zwei angepaßte Positionierungsringe 13, 14 in zusammengeklapptem Zustand derart fixiert, dass der Bereich über dem Probenaufnahmebehälter 1 luftdicht verschlossen ist. Die Ringe 13, 14 sind vorzugsweise derart ausgebildet, dass sie sich von der Oberkante zur Basis hin verjüngen. Dadurch wird gewährleistet, dass die Ringe 13, 14 beim Zusammenfalten von weiblicher Flagge 11 und Probenaufnahmebehälter 1 ineinander einrasten und so dicht aneinander anliegen, dass sie luft- und wasserdicht geschlossen werden können. Die Positionierungsringe 13, 14 gewährleisten zudem, dass der Probenaufnahmebehälter 1 exakt an der richtigen Stelle der weiblichen Flagge 11 angeordnet wird, so dass die Spitze des Dorns 4, 8 beim Einziehen der Ohrmarke genau in den Sammelbehälter gedrückt wird.

Beim Anbringen der Ohrmarke wird dieser Bereich erst beim Durchstoßen der weiblichen Flagge 11 mit der Hohlspitze des Dorns 4 geöffnet. Gleichzeitig wird die ggf. vorhandene, abdeckende Membran/ Folie des Probenaufnahmebehälters 1 durchstossen und die beiden Räume somit kurzfristig verbunden, bis das Probengewinnungsmittel 4 ganz in den Probenaufnalrrnebehälter 1 eingebracht wurde und diesen abdichtet.

Im Prinzip kann in dem von der weiblichen Flagge 11 und dem Probenaufnahmebehälter 1 gebildeten Raum Material eingelagert werden, das bei der Probennahme mit der Probe in Kontakt kommen soll. Es ist somit möglich, zwei voneinander getrennte Räume zum Beladen mit Komponenten zu nutzen, die erst beim Stanzen der Probe zusammengeführt und beispielsweise aktiviert werden sollen. Es ist zudem möglich, auf die Membran, die den Probenaufnahmebehälter 1 verschliesst, künstliche Oligonukleotide aufzubringen, die eine DNA-Codierung der Ohrmarkennummer darstellen. Da dieser Raum gegen äussere Einflüsse geschützt ist, bleiben die Oligonukleotide dort bis zur Benutzung der Ohrmarke stabil. Beim Stanzen der Probe werden diese Oligonukleotide automatisch mit in die Probenkammer transportiert.

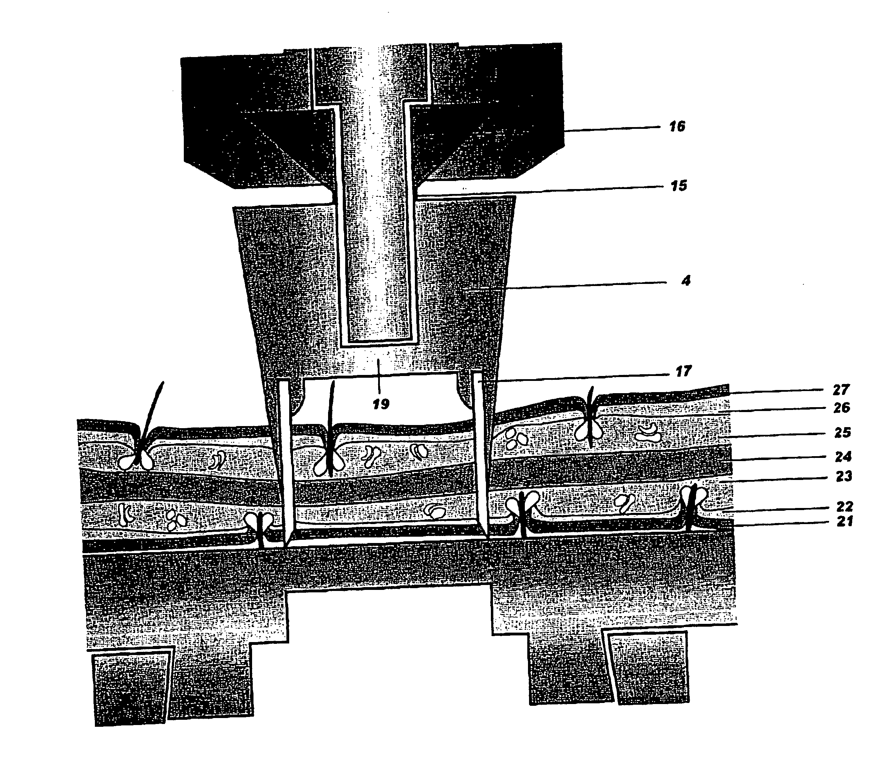

Bei der Anbringung der Ohrmarke wird das gesamte Ohr durch Drücken der Kante der Hohlspitze gegen den Kunststoff der Gegenplatte (siehe Fig. 2) durchstoßen, wobei das ausgestanzte Gewebe in die Hohlspitze gelangt. Anschließend durchdringt der Dorn 4, 8 die weibliche Flagge 11 an der dafür vorgesehenen Stelle, wobei ein Plastikring aus der Flagge ausgestanzt oder ein Teil der Durchtrittstelle aufgetrennt wird, so dass der Dorn 4 durch diese so gebildete Öffnung schlüpfen kann. Auch bei diesen Ohrmarken schliesst der Dorn 4 die Ohrmarke und fixiert die beiden Ohrmarkenteile 10, 11 miteinander.

Um die Stanzwirkung noch zu erhöhen, kann der Bereich der weiblichen Flagge 11, auf den der Dorn 4 mit der Hohlspitze trifft, dieser Spitze leicht entgegengewölbt sein oder eine kleine Nut aufweisen, in die der Stanzkopf beim Durchdrücken eintritt. Weiter kann ein Metallring in die weibliche Flagge 11 eingelegt/eingegossen sein, der für die Metallhülse des Dorns eine Gegenschneide darstellt, so dass es beim Schliessen der Ohrmarke zu einem Vorgang kommt, bei dem zwei Metallschneiden gegeneinander bewegt werden und das dazwischenliegende Gewebe an den scharfen Kanten exakt zerschnitten wird.

Mit der erfindungsgemäßen Ohrmarke kann tatsächlich ein in der Regel sauber rund geformtes Loch aus dem Ohr ausgestanzt werden. Gemäß Untersuchungen mit Gewebestücken, die mit den erfindungsgemäßen Ohrmarken ausgestanzt wurden, sind diese Gewebestücke - beim Einziehen der Ohrmarke von der Ohrinnenseite nach aussen - wie folgt zusammengesetzt:
1. Epidermis (Oberhaut) - Ohrinnenseite
2. Korium (Lederhaut)
3. Subcutis (Unterhaut)
4. Ohrknorpel
5. Subcutis (Unterhaut)
6. Korium (Lederhaut)
7. Epidermis (Oberhaut) - Ohraussenseite

Dieser Aufbau des ausgestanzten Gewebestückes zeigt deutlich, dass beim Einziehen der modifizierten Ohrmarke das gesamte Ohr durchstanzt und von allen vorhandenen Gewebeschichten Material gewonnen wurde.

Ein Beleg für das tatsächlich vollständige Durchstanzen des Ohres liess sich auch daraus gewinnen, dass Ohren, die von geschlachteten Tieren stammten, nach dem Einziehen und dem anschliessenden Entfernen der Ohrmarke saubere, kreisrunde Löcher aufwiesen, die den Durchmesser der verwendeten Hohlspitze hatten. Beim Einziehen der Ohrmarke war also eine Scheibe Ohrgewebe sauber ausgestanzt und aus dem Ohr entfernt worden, so dass sich die Ränder des Loches nach dem Entfernen der Ohrmarke nicht aneinanderlegen liessen, weil das ausgestanzte runde Gewebestück fehlte.

Bei der Verwendung konventioneller Ohrmarken war zwar nach dem Entfernen frisch eingezogener Ohrmarken ebenfalls eine Öffnung im Ohr feststellbar, diese Öffnung war aber kein rundes Loch sondern ein schlitzförmiger Riss, dessen Ränder sich nach dem Entfernen der Ohrmarke wieder aneinander legen liessen, da es anscheinend zu keinem nennenswerten Substanzverlust gekommen war.

Die Einheilung der Ohrmarke kann dadurch noch weiter verbessert werden, dass durch Aufbringen von bakteriostatisch oder bakteriozid wirkenden Substanzen am Dorn der Ohrmarke erreicht wird, dass dieser nach dem Einziehen der Ohrmarke bei den Stanzrändern die Entstehung einer bakteriellen Entzündung verhindert/unterbindet. Da der Dorn sich im Ohrloch frei bewegen kann, wird die Heilungstendenz nicht durch Reib- und Scheuereinwirkungen gestört und es kommt ausserdem zu keinem Sekretstau. Die ablaufenden Vorgänge kommen denen einer komplikationslosen "primären Wundheilung" sehr nahe.

Die hier beschriebenen modifizierten Ohrmarken können im Prinzip mit allen vorhandenen konventionellen Ohrmarkenzangen verschiedenster Hersteller eingezogen werden. Gegebenenfalls sind lediglich zur Aufnahme der Probenaufnahmebehälter 1 kleinere technische Änderungen erforderlich.

Die Hohlspitze des Dorns wird so geformt, dass ihre Basis, die über eine Sollbruch/Lösestelle mit dem eigentlichen Dorn . verbunden ist, den Probenaufnahmebehälter 1 abdichtend verschliesst. Gleichzeitig kann der Zwischenboden so ausgelegt werden, dass er dünn genug ist, um im Labor beim Gewinnen der Probe aus dem Sammelbehälter durchbrochen zu werden. Das Gewinnen der Probe aus dem Sammelbehälter kann so von statten gehen, dass in einem ersten Schritt der gesamte Kopf des Dorns, der sich mit der Probe im Sammelbehälter befindet, durch einen Stift, der in der Innenbohrung des Dornkopfes Platz findet, einige Millimeter durch den Boden des Sammelbehälters gedrückt wird. Der Kunststoffboden des Sammelbehälters reisst dabei bereits ein, aber es kommt noch zu keinem Herausdrücken der Probe, weil der Zwischenboden noch intakt ist.

Nunmehr wird der ausserhalb der Hülse gelegene Teil des Dornkopfes durch eine ringförmige Vorrichtung fixiert bzw. zurückgehalten. Unter weiterem Druck durch den in der Innenbohrung befindlichen Stift auf den Zwischenboden wird dieser Zwischenboden ausgebrochen und dadurch die Probe in ein unter dem Sammelbehälter befindliches Gefäss transportiert. In diesem Gefäss kann bereits Material (Enzymlösungen) zum Verdau der Gewebeprobe vorbereitet sein. Nach dem Verdau der Gewebeprobe wird ein Aliquot dieser Lösung durch eine durch die Innenbohrung des Dornkopfes geschobene Kanüle oder Pipettenspitze aufgenommen und z. B. in eine 96-Well-Platte übertragen. In einem weiteren Pipettierschritt in das Gefäss des Probenbehälters 1 kann die Wirkung der Enzymlösung durch bestimmte einpipettierte Substanzen gestoppt werden und weiters Substanzen zugegeben werden, die die DNA für die Lagerung vorbereiten.

Die hintere Öffnung des Probenaufnahmebehälters 1 kann entweder mit einer Kunststofffolie zugeschweisst oder mit einer dicht schliessenden Kappe abgedeckt werden. Der Probenaufnahmebehälter 1, der die Identifikationsnummer des Tieres trägt und auch einen elektronischen Chip und künstliche Oligonukleotide, die die Nummer kodifizieren, enthalten kann, kann als Rückstellprobe oder als Teil einer genomischen DNA-Bank eingelagert werden. Dadurch bleibt die Möglichkeit, auch nach einer Lagerung von mehreren Jahren noch einen zweifelsfreien und fehlerfreien Zugriff auf die Gewebe/DNA-Probe aller einmal markierten Tiere zu haben.

### Beispiel 1:

### Gewinnung von DNA-Proben bei Lämmern

Bei 40 Milchschaf-Lämmern wurden in den ersten Lebenstagen Proben mittels Ohrmarken aus den Ohren gewonnen. Bei allen 40 Ohrmarken, die eine geschlossene weibliche Flagge und einen Dom-Hohlspitzen-Durchmesser von 3,2 mm aufwiesen, waren komplette Gewebestücke mit Haut von der Innen- und Aussenseite sowie Knorpel des Ohres im Sammelbehälter enthalten. Bei der Isolation von DNA aus diesen Gewebestücken mit einem Kit von Machery & Nagel oder mit der Säulchenmethode von Mira wurden mehr als 20 µg hochmolekulare DNA isoliert.

### Beispiel 2:

### Gewinnung von DNA-Proben bei adulten Rindern

Bei Ohren adulter geschlachteter Rinder (älter als 18 Monate) wurden mit modifizierten Ohrmarken, die einen dem Dorndurchmesser entsprechenden Hohlspitzendurchmesser von 5 mm aufwiesen, Gewebeproben entnommen. In allen Fällen konnte eine komplette Gewebeprobe (2x Haut und Unterhaut und 1x Knorpel) aus dem Probenaufnahmebehälter 1 entnommen werden. Die Ohrmarken waren nach dem Einziehen in dem ausgestanzten Loch frei beweglich. Die aus diesen Geweben isolierte DNA-Menge betrug in allen Fällen mehr als 100 µg hochmolekulare DNA. Beim Entfernen der Ohrmarken konnte festgestellt werden, dass in allen Fällen ein rundes Loch (ø 5 mm) aus dem Ohr ausgestanzt worden war.

## Patentansprüche

1. Ohrmarke, umfassend eine Dornplatte (10), eine Gegenplatte (11) sowie einen Probenaufnahmebehälter (1) und Mittel zum Gewinnen der Probe (4), das nach Gewinnen der Probe in den Probenaufnahmebehälter eingeführt wird und diesen dichtend verschließt, wobei
das Mittel zum Gewinnen der Probe (4) eine lösbare Hohlspitze aufweist und **dadurch gekennzeichnet, daß** die Gegenplatte (11) keine zur Durchführung des Dorns ausgebildete Durchgangsöffnung besitzt.

2. Ohrmarke nach Anspruch 1, **dadurch gekennzeichnet, daß** der Schneiderand der Hohlspitze einen wellenförmigen Verlauf aufweist.

3. Ohrmarke nach Anspruch 1, **dadurch gekennzeichnet, daß** in der Hohlspitze ein von außen identifizierbares Kennzeichnungsmittel vorgesehen ist.

4. Ohrmarke nach einem der vorhergehenden Ansprüche, **dadurch gekennzeichnet, daß** auf der Gegenplatte (11) an der Stelle, an der der Dorn (8) durchtreten soll eine Markierung vorgesehen ist.

5. Ohrmarke nach einem der vorhergehenden Ansprüche, **dadurch gekennzeichnet, daß** der Probenaufnahmebehälter (1) luft- und wasserdicht abgeschlossen ist.

6. Ohrmarke nach Anspruch 5, worin die Abdichtung eine Membran ist, auf der Oligonukleotide aufgebracht sind, die zur Kennzeichnung der gewonnenen Probe verwendet werden.

7. Verwendung einer Ohrmarke nach einem der vorhergehenden Ansprüche zur Gewinnung DNA-haltigem biologischem Material und gleichzeitiger Kennzeichnung des Individuums, von dem das biologische Material gewonnen wurde.

8. Verwendung nach Anspruch 7, wobei das Individuum ein Nutztier ist, wie Rind, Schwein oder Schaf.

9. Verwendung nach Anspruch 7 oder 8, wobei der Probensammelbehälter nach Anbringung der Ohrmarke am Tier verbleibt.

10. Verfahren zur Kennzeichnung von Tieren mit gleichzeitiger Gewinnung biologischer Proben davon, wobei eine Ohrmarke gemäß einem der Ansprüche 1 bis 6 eingesetzt wird.

## Claims

1. Ear tag comprising a spike plate (10), a counter plate (11) as well as a sample collecting container (1) and means for collecting the sample (4), which after collecting the sample is introduced into the sample collecting container and tightly sealing it, wherein the means for collecting the sample (4) has a releasable hollow tip and **characterized in that** the counter plate (11) has no through hole formed for transition of the spike.

2. Ear tag according to claim 1, **characterized in that** the cutting edge of the hollow tip has an undulated form.

3. Ear tag according to claim 1, **characterized in that** a **characterizing** means identifiable from outside is provided in the hollow tip.

4. Ear tag according to any of the preceding claims, **characterized in that** a marking is provided at the place where the spike (8) shall transit the counter plate (11).

5. Ear tag according to any of the preceding claims, **characterized in that** the sample collecting container (1) is sealed in an air- and water-proof manner.

6. Ear tag according to claim 5, wherein the seal is a membrane on which oligonucleotides are applied which are used for **characterizing** the collected sample.

7. Use of an ear tag according to any of the preceding claims, for collecting DNA-containing biological material and simultaneously **characterizing** the individual from which the biological sample was collected.

8. The use according to claim 7, wherein the individual is livestock such as cattle, pig or sheep.

9. The use according to claim 7 or 8, wherein after fixing the ear tag, the sample collecting container remains on the animal.

10. Method for identifying animals and simultaneously collecting biological samples therefrom, wherein an ear tag according to any of the claims 1 to 6 is used.

## Revendications

1. Marque auriculaire comprenant une plaque munie d'une broche (10), une contreplaque (11), un moyen de prélèvement d'échantillon (4) qui, après prélèvement dudit échantillon, est introduit dans le récipient pour échantillon et obture ce dernier de manière étanche, et dans laquelle le moyen de prélèvement d'échantillon (4) comporte une pointe creuse, et **caractérisée en ce que** la contreplaque (11) est dépourvue d'ouverture pour le passage de ladite broche.

2. Marque auriculaire selon la revendication 1, **caractérisée en ce que** le bord de coupe de la pointe creuse est ondulé.

3. Marque auriculaire selon la revendication 1, **caractérisée en ce que** qu'une marque de reconnaissance identifiable de l'extérieur est disposée à l'intérieur de la pointe creuse.

4. Marque auriculaire selon l'une des revendications précédentes, **caractérisée en ce qu'**un repère est disposé sur la contreplaque (11) à l'endroit prévu pour le passage de la broche(8).

5. Marque auriculaire selon l'une des revendications précédentes, **caractérisée en ce que** le récipient pour échantillon (1) est obturé de manière étanche à l'air et à l'eau.

6. Marque auriculaire selon la revendication 5 dans laquelle le moyen d'étanchéité est une membrane sur laquelle ont été déposés des oligo-nucléotides destinés à l'identification de l'échantillon.

7. Utilisation d'une marque auriculaire selon l'une des revendications précédentes pour le prélèvement d'échantillon de matériel biologique contenant des ADN et le marquage simultané de l'individu sur lequel l'échantillon a été prélevé.

8. Utilisation selon la revendication 7 dans laquelle l'individu est un bétail tel le boeuf, le porc ou le mouton.

9. Utilisation selon l'une des revendications 7 et 8 dans laquelle l'échantillon de matériel biologique subsiste sur l'animal après la pose de la marque auriculaire.

10. Procédé de marquage d'animaux et de prélèvement simultané de matériel biologique à partir de ceux-ci par la mise en place d'une marque auriculaire selon l'une des revendications 1 à 6.
